# EUROPÄISCHE PATENTSCHRIFT

(11) **EP 1 071 643 B1**
(45) Veröffentlichungstag und Bekanntmachung des Hinweises auf die Patenterteilung: **10.09.2003**
(21) Anmeldenummer: 98966260.6
(22) Anmeldetag: 08.12.1998
(51) Int. Cl.: C07C 37/20, C07C 39/16, B01J 8/06

(54) **VERFAHREN ZUR HERSTELLUNG VON BIS(4-HYDROXYARYL)ALKANEN**
METHOD FOR PRODUCING BIS(4-HYDROXYARYL)ALKANES
PROCEDE POUR PREPARER DES BIS(4-HYDROXYARYL)ALCANES

(30) Priorität: 19.12.1997 DE 19756771
(43) Veröffentlichungstag der Anmeldung: 31.01.2001
(73) Patentinhaber: BAYER AG, 51368 Leverkusen (DE)
(72) Erfinder: EEK, Rob, D-51061 Köln (DE); HALLENBERGER, Kaspar, D-51375 Leverkusen (DE); MENDOZA-FROHN, Christine, D-40699 Erkrath (DE); RONGE, Georg, D-40227 Düsseldorf (DE); FENNHOFF, Gerhard, D-47877 Willich (DE); SLUYTS, Domien, B-2940 Stabroek (BE); VERHOEVEN, Werner, B-2920 Kalmthout (BE)
(86) Internationale Anmeldenummer: EP9807973
(87) Internationale Veröffentlichungsnummer: WO99032423

(56) Entgegenhaltungen:
- EP-A- 0 754 666
- WO-A-94/19079

## Beschreibung

Die vorliegende Erfindung betrifft ein Verfahren zur Herstellung von Bis(4-hydroxyaryl)alkanen durch sauer katalysierte Umsetzung aromatischer Hydroxyverbindungen mit Ketonen, bei dem man Temperatur, Keton- und Wassergehalt einer flüssigen Reaktionsmischung mittels einer Gasphase geeigneter Zusammensetzung einstellt.

Die Synthese von Bis(4-hydroxyaryl)alkanen durch sauer katalysierte Umsetzung aromatischer Hydroxyverbindungen mit Ketonen ist bekannt. Aus US-A 3 634 341 und US-A 3 760 006 geht hervor, daß der Wassergehalt des Reaktionsgemischs die Katalysatoreffizienz beeinflußt, als kritische Grenze werden für die Umsetzung im Festbettreaktor 2-3 Gew.-% Wassergehalt angegeben. Aus WO 94/19079 und EP-A 770 590 ist bekannt, das bei der Reaktion gebildete Wasser kontinuierlich zu entfernen, indem ein trockener Inertgas, z.B. Stickstoff, im Gegenstrom durch den Reaktor geleitet wird. Aus US-A 4,400,555 (EP-A 342 758) und EP-A 754 666 geht hervor, daß sich in einem Herstellungsverfahren mit mehreren hintereinander geschalteten Reaktoren die Selektivität der Bildung von Bis(4-hydroxyaryl)alkan steigern läßt, wenn das Keton auf die verschiedenen Reaktoren verteilt wird; in WO 94/19079 wird vorgeschlagen, bei der Herstellung von Bis(4-hydroxyaryl)alkan in einer (Stripp-)Kolonne das Keton gasförmig oder flüssig über mehrere über die Gesamtlänge der Kolonne verteilte Dosierstellen zuzuführen. Der apparative und regelungstechnische Aufwand einer solchen Aufteilung der Ketonzufuhr auf mehrere Reaktoren bzw. mehrere Zufuhrstellen eines Strippers ist ein Nachteil dieser Verfahren. Außerdem sinkt durch eine Aufteilung der Ketonzufuhr die Reaktionsgeschwindigkeit der Bildung von Bis(4-hydroxyaryl)alkan. Die geringere Reaktionsgeschwindigkeit wird in Kauf genommen, um eine Erhöhung der Selektivität zu erzielen.

Wünschenswert ist eine Verfahrensweise, bei der nicht nur eine höhere Selektivität der Reaktion, sondern auch gleichzeitig eine höhere Reaktionsgeschwindigkeit erzielt werden, um so die Raum-Zeit-Ausbeute zu maximieren.

Es wurde nun gefunden, daß sich Bis(4-hydroxyaryl)alkane mit hoher Selektivität und gleichzeitig in hoher Raum-Zeit-Ausbeute herstellen lassen, wenn man Temperatur, Keton- und Wassergehalt einer flüssigen Reaktionsmischung mittels einer Gasphase geeigneter Zusammensetzung einstellt.

Gegenstand der Erfindung ist ein Verfahren zur Herstellung von Bis(4-hydroxyaryl)alkanen durch sauer katalysierte Umsetzung aromatischer Hydroxyverbindungen mit Ketonen, bei dem eine Flüssigphase, die aromatische Hydroxyverbindung, Keton und gegebenenfalls Wasser enthält, und eine Gasphase, die aromatische Hydroxyverbindung, Keton und gegebenenfalls Wasser in solchen Konzentrationen enthält, daß im Reaktor Wasser aus der Flüssigphase in die Gasphase und Keton aus der Gasphase in die Flüssigphase übergehen, durch einen Reaktor geleitet werden.

Gas- und Flüssigphase können im Gleich- oder Gegenstrom durch den Reaktor geleitet werden. Bevorzugt ist die Gegenstrom-Fahrweise.

Durch das erfindungsgemäße Verfahren lassen sich über die gesamte Reaktorlänge frei wählbare, annähernd konstante Keton- und Wasserkonzentrationen bei gleichzeitig annähernd konstanten, frei wählbaren Temperaturen einstellen. Es kann erfindungsgemäß ebenfalls eine Fahrweise durchgeführt werden, bei der anstelle annähernd konstanter Bedingungen solche gewählt werden, bei denen die Temperatur und/oder die Konzentrationen an aromatischer Hydroxyverbindung, Keton und Wasser in der Flüssigphase nicht über die gesamte Länge des Reaktor konstant sind. So können beispielsweise Temperaturgradienten und/oder Konzentrationsgradienten für Keton und/oder Wasser in der flüssigen Phase aufgeprägt werden. Grundgedanke des erfindungsgemäßen Verfahrens ist die Einstellung der Temperatur, des Keton- und Wassergehalts in der flüssigen Reaktionsmischung mittels einer Gasphase geeigneter Zusammensetzung.

Im erfindungsgemäßen Verfahren beträgt das Molverhältnis von aromatischer Hydroxyverbindung zu Keton in der dem Reaktor zugeführten Flüssigphase im allgemeinen 3,5:1 bis 125:1, bevorzugt 5:1 bis 60:1, besonders bevorzugt 10:1 bis 30:1. Die dem Reaktor zugeführte Flüssigphase kann geringe Mengen Wasser enthalten. Bevorzugt beträgt der Wassergehalt 0 bis 2 Gew.-%.

Das Molverhältnis von aromatischer Hydroxyverbindung zu Keton in der dem Reaktor zugeführten Gasphase ergibt sich aus der Bedingung, daß im Reaktor bei Reaktionstemperatur und Reaktionsdruck Keton aus der Gasphase in die Flüssigphase übergehen soll. Dementsprechend ist für die Einstellung annähernd konstanter Ketonkonzentrationen in der Flüssigphase die Konzentration an Keton in der Gasphase am Reaktoreintritt ungefähr so hoch zu wählen wie die Konzentration, die sich bei Reaktionstemperatur und Reaktionsdruck im thermodynamischen Gleichgewicht über der zudosierten Flüssigphase in der Gasphase einstellen würde. Soll im Reaktor ein Gradient der Ketonkonzentration eingestellt werden, so ist die Ketonkonzentration in der dem Reaktor zugeführten Gasphase höher oder tiefer zu wählen als die Konzentration, die sich im thermodynamischen Gleichgewicht einstellen würde.

Die Konzentration an Wasser in der zudosierten Gasphase ergibt sich nach analogem Prinzip aus der Wasserkonzentration in der zudosierten Flüssigphase. Sie muß so gewählt werden, daß im Reaktor bei Reaktionstemperatur und -druck Wasser aus der Flüssigphase in die Gasphase übergeht. Dementsprechend ist die Konzentration an Wasser in der Gasphase ungefähr so hoch zu wählen wie die Konzentration, die sich bei Reaktionstemperatur und -druck im thermodynamischen Gleichgewicht über der zudosierten Flüssigphase in der Gasphase einstellen würde, um eine konstante Wasserkonzentration in der Flüssigphase einzustellen. Soll im Reaktor ein Gradient der Wasserkonzentration eingestellt werden, so ist die Wasserkonzentration in der dem Reaktor zugeführten Gasphase höher oder tiefer zu wählen als die Konzentration, die sich im thermodynamischen Gleichgewicht einstellen würde.

Durch geeignete Einstellung der Konzentrationen in Gas- und Flüssigphase werden im Reaktor der flüssigen Reaktionsmischung durch die Gasphase kontinuierlich Wasser entzogen und Keton zugeführt. Der Verbrauch von Keton bzw. die Entstehung von Wasser durch Reaktion werden somit durch den Phasenübergang zwischen Gas- und Flüssigphase ausgeglichen. Auf diese Weise können die Konzentrationen von Keton und Wasser in der Flüssigphase sowie die Temperatur im Reaktor annähernd konstant gehalten werden oder ein Konzentrations- oder Temperaturgradient aufgeprägt werden.

Diese Fahrweise ist sehr vorteilhaft, da eine ständige Nachdosierung von verbrauchtem Keton und die gleichzeitig erfolgende kontinuierliche Entfernung des Wassers aus der flüssigen Phase die Reaktionsgeschwindigkeit über die gesamte Länge des Reaktors hoch erhält. Die kontrollierbaren, gegebenenfalls annähernd konstanten Reaktionsbedingungen im Reaktor, sowie die nahezu isotherme Fahrweise, ermöglichen gleichzeitig eine Verbesserung der Selektivität.

Die Temperatur im Reaktor liegt zwischen der Kristallisationstemperatur der Reaktionsmischung und 130°C. Sie wird durch die Wahl des Drucks im Reaktor eingestellt. Werden Keton- und Wasserkonzentration über die gesamte Länge des Reaktors konstant gehalten, läßt sich eine sehr gleichmäßige Temperaturführung erreichen. Die Wärmeabfuhr und -zufuhr erfolgt im Gegensatz zum dem in WO 94/19079 beschriebenen Verfahren nicht durch die Erwärmung eines Inertgasstroms, sondern viel effektiver hauptsächlich durch lokale Verdampfung und Kondensation. Im Gegensatz zu den Verfahren des Standes der Technik kann so die Temperatur über die gesamte Reaktionszone annähernd konstant gehalten werden.

Der einzustellende Druck läßt sich für die gewünschte Temperatur und die gewünschten Keton- und Wasserkonzentrationen über das thermodynamische Gleichgewicht berechnen; er beträgt bei inertgasfreier Fahrweise im allgemeinen 5 bis 1000 mbar, bevorzugt 15 bis 200 mbar. Zur Einstellung der Temperatur im Reaktor über die Vorgabe des Drucks kann gegebenenfalls dem Gaszulauf ein Inertgas wie Stickstoff zugegeben werden.

Soll ein Temperaturgradient im Reaktor eingestellt werden, z.B. um eine Kristallisation des mit Bis(4-hydroxyaryl)alkan angereicherten Reaktionsgemischs im unteren Teil des Reaktors zu vermeiden, kann durch die Erzeugung von Druckstufen im Reaktor oder die Aufprägung eines Konzentrationsgradienten die Temperatur im Reaktor und darüber hinaus - über die Keton- und Wasserkonzentration in der Flüssigphase - die Kristallisationstemperatur der Reaktionsmischung beeinflußt werden.

Die Temperatur der zugeführten Flüssigphase sollte der Siedetemperatur bei dem im Reaktor vorgegebenen Druck entsprechen, kann aber auch darunter liegen, die Temperatur der zugeführten Gasphase sollte der Tautemperatur bei dem im Reaktor vorgegebenen Druck entsprechen oder geringfügig darüber liegen.

Selbstverständlich können bei der Ausführung des erfindungsgemäßen Verfahrens durch zusätzliche Dosierstellen von Gas- bzw. Flüssigzulauf oder durch Maßnahmen, die lokale Druckveränderungen im Reaktor bewirken, gezielt Temperatur- bzw. Konzentrationsprofile und/oder Temperatur- bzw. Konzentrationsstufen ausgeführt werden.

Geeignete saure Katalysatoren zur Herstellung von Bis(4-hydroxyaryl)alkanen sind dem Fachmann bekannt. Es können homogene Katalysatoren sein, z.B. Salzsäure, Schwefelsäure, Bortrifluorid etc. Homogene Katalysatoren können über eine separate Dosierung und/oder gemeinsam mit der Flüssigphase dosiert werden. Bevorzugt werden heterogene Katalysatoren eingesetzt. Als heterogene Katalysatoren werden dem Fachmann bekannte Katalysatoren eingesetzt, beispielsweiset saure lonentauscherharze, feste anorganische Säuren, funktionalisierte anorganische feste Säuren usw. Bevorzugt werden Katalysatoren mit Sulfonsäuregruppen in organischer, anorganischer oder einer Kombination aus organischer und anorganischer Matrix verwendet. Die heterogenen Katalysatoren werden bevorzugt gemeinsam mit schwefelhaltigen Cokatalysatoren eingesetzt. Solche Cokatalysatoren sind dem Fachmann bekannt. Sie können gasförmig, gegebenenfalls zusammen mit dem Eduktgasstrom (z.B im Falle von H₂S) oder homogen zusammen mit der Flüssigphase dosiert oder vorab auf einem heterogenen Katalysator fixiert werden Beispiele für solche Katalysatoren gehen aus US-A 2 468 982, US-A 2 623 908, US-A 2 775 620, US-A 3 634 341, US-A 3 760 006, DE-OS 36 19 450 oder DE-OS 37 27 641 hervor.

Als Reaktoren können diejenigen, dem Fachmann bekannten Reaktoren verwendet werden, die einen intensiven Stoffaustausch zwischen Gas- und Flüssigphase und gleichzeitig einen intensiven Kontakt der Flüssigphase mit einem heterogenen Katalysator ermöglichen. Dies sind beispielsweise Mehrphasenreaktoren mit suspendiertem Katalysator (begaster Rührkessel, Blasensäule, Dreiphasenwirbelschicht usw.), Mehrphasenreaktoren mit fest angeordnetem Katalysator (z.B. Rieselbetten) oder Rektifikationskolonnen unterschiedlicher Ausführung. Bevorzugt wird als Reaktor eine Reaktivrektifikationskolonne verwendet. Sie wird in der dem Fachmann bekannten Weise als Bodenkolonne oder Packungskolonne ausgeführt. In der bevorzugten Verfahrensform mit heterogenem Katalysator wird dieser in der dem Fachmann bekannten Weise in die Bodenkolonne bzw. Kolonne mit geordneter oder ungeordneter Packung eingesetzt. Beispiele sind beschrieben in EP-A 670 178; EP-A 461 855; US-A 5 026 459; US-A 4 536 373; WO 94/08681; WO 94/08682; WO 94/08679; EP-A 470 655; WO 97/26971; US-A 5 308 451; EP-A 755 706; EP-A 781 829, EP-A 428 265; EP-A 448 884; EP-A 640 385; EP-A 631 813; WO 90/02603; WO 97/24174; EP-A 665 041, EP-A 458 472; EP-A 476 938, deutsches Gebrauchsmuster 298 07 007.3.

Alternativ kann der Katalysator in externe, gegebenenfalls temperierte Reaktoren eingebracht werden, wobei die Flüssigphase von der Kolonne in den Reaktor und von dort zur Stofftrennung wieder zurück in die Kolonne geführt wird. Zur Entkopplung der Temperaturen in Kolonne und externen Reaktoren ist eine Temperierung der Ströme zwischen der Kolonne und den externen Reaktoren möglich.

In einer bevorzugten Verfahrensform, bei der als Reaktor eine Reaktivrektifikationskolonne verwendet wird, werden im oberen Teil der Kolonne, oberhalb der Reaktionszone Keton, aromatische Hydroxyverbindung und ggf. Wasser im gewünschten Verhältnis flüssig zudosiert. Durch gleichzeitiges Einspeisen eines Gaszulaufes geeigneter Zusammensetzung im unteren Bereich der Kolonne, unterhalb der Reaktionszone, wird erreicht, daß das Wasser, das in der Flüssigphase durch Reaktion entsteht, kontinuierlich über die Gasphase abtransportiert wird, und daß das durch die Reaktion verbrauchte Keton kontinuierlich in die Flüssigphase nachgeliefert wird. Wahlweise kann im Reaktor eine annähernd konstante Keton- und Wasserkonzentration, oder, beispielsweise zur Vermeidung einer Kristallisation der Reaktionsmischung im unteren Teil der Reaktivrektifikationskolonne, ein Temperaturgradient und/oder ein Gradient der Keton- und/oder Wasserkonzentration eingestellt werden. Bei der Reaktivrektifikation können Konzentrations- und/oder. Temperaturprofile durch die Zusammensetzung der Flüssigphase und einen gegebenenfalls bewußt aufgeprägten Druckverlust und/oder durch zusätzliche Dosierstellen für Gas und/oder Flüssigkeit eingestellt werden. Wird die Ketonkonzentration im unteren Teil der Kolonne, wo in der Reaktionsmischung ein hoher Gehalt an Bis(4-hydroxyaryl)alkan vorliegt, erhöht, so weist die Reaktionsmischung einen niedrigeren Kristallisationspunkt auf, so daß die Reaktionstemperatur niedrigerer gewählt werden kann.

Über gegebenenfalls eingebaute Wärmetauscher kann Wärme dem Reaktor zugeführt oder aus ihrn abgeführt werden. Einfacher ist die Fahrweise ohne Wärmetauscher. Der Stoffaustausch zwischen Gas- und Flüssigphase innerhalb des Reaktors wird in der dem Fachmann bekannten Weise optimiert. Bei der bevorzugten Ausführung als Reaktivrektifikation wird die installierte Trennleistung in Abhängigkeit vom Reaktionsfortschritt so gewählt, daß das verbrauchte Keton ausreichend schnell von der Gas- in die Flüssigphase nachgeführt und das entstehende Wasser ausreichend schnell von der Flüssig- in die Gasphase transportiert wird. Bevorzugt werden dazu dem Fachmann bekannte Packungen oder Böden eingesetzt. Bei Verwendung eines heterogenen Katalysators wird dieser in dem Fachmann bekannter Weise auf den Böden bzw. in der Packung angeordnet. Bei Verwendung eines homogenen Katalysators und/oder Cokatalysators kann dieser mit der Flüssigdosierung und/oder über zusätzliche Dosierstellen in den Reaktor dosiert werden. Bei Verwendung eines gasförmigen Cokatalysators kann dieser mit der Gasdosierung und/oder über zusätzliche Dosierstellen in den Reaktor gebracht werden.

Unterhalb der Reaktionszone können sich eine Destillationszone und ein Verdampfer befinden. Dadurch können aus dem Flüssigablauf Keton, Wasser und Teile der aromatischen Hydroxyverbindung und eventuell Cokatalysator verdampft werden, so daß nur ein Teil des Gasstroms extern zugeführt werden muß. Der diese Destillationszone verlassende Produktstrom weist verringerte Konzentrationen an Keton und Wasser und eine erhöhte Konzentration an Bis(4-hydroxyaryl)alkan auf.

Der Produktstrom kann in dem Fachmann bekannter Weise aufgearbeitet werden. So kann aus dem Produktstrom das Bis(4-hydroxyaryl)alkan beispielsweise durch Kristallisation (siehe z.B. DE-OS 42 13 872, EP-A 671 377 bzw. US-A 5 545 764), Extraktion mit einem organischen Lösungsmittel, Destillation (siehe z.B. US-A 5 785 823) oder eine Kombination dieser Maßnahmen isoliert werden.

Der Gasstrom, der oben aus der Reaktionszone austritt, wird, gegebenenfalls in Verbindung mit vollständiger oder teilweise Verdichtung, nach im Prinzip bekannten Methoden zur Stofftrennung in seine Komponenten oder Gemische von Komponenten zerlegt. Die so gewonnenen Teilströme können dann flüssig oder gasförmig dem Verfahren wieder zugeführt werden, wobei jedoch das gebildete Reaktionswasser aus dem Verfahren ausgeschleust wird. Zur Stofftrennung können die dem Fachmann bekannten Methoden eingesetzt werden, z.B. Teilkondensation, Destillation, Extraktion, Membranverfahren usw. Gasförmige Teilströme können ohne weitere Aufarbeitung nach der Verdichtung direkt gasförmig der Kolonne wieder zugeführt werden.

Das Verhältnis der Dosiermengen von Flüssig- und Gasphase wird in der dem Fachmann bekannten Weise vom gewählten Reaktortyp abhängen. Beispielsweise wird bei der Reaktivrektifikation dieses Verhältnis unter Beachtung der durch die Einbauten gegebenen hydrodynamischen Grenzen so gewählt, daß ein effektiver Stoffaustausch gewährleistet ist, keine Ungleichverteilung im Reaktor und außerdem kein Fluten bei hoher Belastung oder Durchregnen bei niedriger Belastung des Reaktors auftritt.

Die Wahl des Mengenverhältnisses von Gas- und Flüssigkeitsstrom beeinflußt auch, inwieweit die Reaktionsbedingungen im Reaktor konstant gehalten werden können. Durch die Abgabe von Keton an die Flüssigphase und die Aufnahme von Wasser aus der Flüssigphase ändert der Gasstrom beim Durchströmen des Reaktors seine Zusammensetzung. Dadurch ergibt sich eine geringfügige Abweichung von den gewünschten konstanten Reaktionsbedingungen. Diese Abweichung ist umso kleiner, je größer das Mengenverhältnis von Gas- und Flüssigkeitsstrom ist; gleichzeitig steigt aber mit steigender Gasmenge der Aufwand für die Recyclisierung des Gasstroms. Als günstig haben sich Verhältnisse von Gas- und Flüssigkeitsstrom von 0,01 bis 10, insbesondere von 0,05 bis 2 erwiesen.

Der dem Reaktor zugeführte Flüssigkeitsstrom setzt sich zusammen aus Edukten, die frisch dem Verfahren zugeführt und/oder im Kreis geführt werden sowie gegebenenfalls dem Rücklauf aus einer partiellen Kondensation des Gasstromes. Der dem Reaktor zugeführte Gasstrom setzt sich zusammen aus Komponenten, die frisch dem Verfahren zugeführt und/oder im Kreis geführt werden sowie gegebenenfalls einem durch teilweise Verdampfung des Flüssigkeitsstroms gewonnenen Anteil. Zwischen Verdampfer und Reaktionsteil kann ein zur besseren Rückführung leichtersiedender Komponenten dienender Destillationsteil installiert werden.

Geeignete aromatische Hydroxyverbindungen für das erfindungsgemäße Verfahren sind in p-Position nicht substituiert und enthalten keine Substituenten zweiter Ordnung wie Cyano-, Carboxy- oder Nitrogruppen; genannt seien beispielsweise Phenol, o- und m-Kresol, 2,6-Dimethylphenol, o-tert.-Butylphenol, 2-Methyl-6-tert.-Butylphenol, o-Cyclohexylphenol, o-Phenylphenol, o-Isopropylphenol, 2-Methyl-6-cyclopentylphenol, o- und m-Chlorphenol, 2,3,6-Trimethylphenol. Bevorzugt sind Phenol, o- und m-Kresol, 2,6-Dimethylphenol, o-tert.-Butylphenol und o-Phenyl-phenol; besonders bevorzugt ist Phenol.

Im erfindungsgemäßen Verfahren können Ketone mit aliphatischen, araliphatischen oder aromatischen Gruppen an der Carbonylfunktion eingesetzt werden; beispielsweise auch ω-Phenylacetophenon oder Benzophenon.

Bevorzugt werden Ketone eingesetzt, die wenigstens eine aliphatische Gruppe an der Carbonylfunktion enthalten; genannt seien beispielsweise Aceton, Methylethylketon, Methylpropylketon, Methylisopropylketon, Diethylketon, Acetophenon, Cyclohexanon, Cyclopentanon, Methyl-, Dimethyl- und Trimethylcyclohexanone, die auch geminale Methylgruppen aufweisen können wie 3,3-Dimethyl-5-methylcyclohexanon (Hydroisophoron). Bevorzugt sind Aceton, Acetophenon, Cyclohexanon und dessen Methylgruppen tragende Homologe; besonders bevorzugt ist Aceton.

Geeignete Edukte für das erfindungsgemäße Verfahren sind auch die nach Abtrennung der Bis(4-hydroxyaryl)alkane verbleibenden Mutterlaugen, die nach Zugabe der verbrauchten Hydroxyverbindungen und gegebenenfalls Entnahme eines gewissen Anteils zur Vermeidung der Anreicherung unerwünschter Beiprodukte in das Verfahren zurückgeführt werden; für den Fall der Synthese von Bisphenol A enthalten solche Mutterlaugen ca. 75-90 Gew.-% Phenol und 10-25 Gew.-% Beiprodukte, die sich wie folgt zusammensetzen:

| | |
|---|---|
| Bisphenol A | 25 - 80 Gew.-% |
| o,p-Bisphenol | 5 - 20 Gew.-% |
| Trisphenol | 2 - 10 Gew.-% |
| Chromane | 2 - 20 Gew.-% |
| 1,3,3-Trimethyldihydroxyphenylindane | 1 - 15 Gew.-% |
| weitere Nebenprodukte | 0,5 - 10 Gew.-% |

Beim erfindungsgemäßen Verfahren, insbesondere bei dessen Ausführung in einer Reaktionsrektifikationskolonne, werden durch die kontinuierliche und effiziente Abtrennung des Reaktionswassers, das durch Blockierung der Katalysatoroberfläche die Reaktion hemmen kann, und die kontinuierliche Nachdosierung des Ketons höhere Reaktionsgeschwindigkeiten und damit größere Raum-Zeit-Ausbeuten erreicht. Gleichzeitig wird eine sehr konstante Temperaturführung möglich, da im System auftretende exo- oder endotherme Effekte nicht zu einer Temperaturänderung führen, sondern durch partielle Verdampfung oder Kondensation kompensiert werden. Aufgrund der geringeren Verweilzeit im Reaktor und der Kontrolle von Temperatur, Wasser- und Ketongehalt der Flüssigphase im Reaktor wird eine erhöhte Selektivität der Reaktion und damit eine Verringerung der Nebenproduktbildung und eine Erhöhung der Produktqualität beobachtet. Dies sind auch Bedingungen, die die Standzeit der Katalysatoren verbessern.

Es ist selbstverständlich auch eine Kombination des erfindungsgemäßen Verfahrens mit klassischer Reaktionstechnik für die Herstellung von Bis(4-hydroxyaryl)alkanen denkbar. So kann man beispielsweise einen klassischen Festbettreaktor, in dem man den Anfangsumsatz des Ketons durchführt, vor das erfindungsgemäße Verfahren schalten, in dem man den Restumsatz zur Vervollständigung der Reaktion durchführt. Der Anfangsumsatz im Festbettreaktor bei hoher Ketonkonzentration, niedriger Wasserkonzentration und niedriger Anfangstemperatur führt zu einer hohen Raumzeitausbeute und guter Produktqualität im Rohprodukt, welches als Flüssigphase in das erfindungsgemäße Verfahren dosiert wird, Hier kann bei den vorteilhaften Bedingungen des erfindungsgemäßen Verfahrens, wie beispielsweise annähernd konstant niedrige Wasserkonzentration und/oder hohe Ketonkonzentration bzw. annähernd konstante Temperatur der Restumsatz durchgeführt werden.

Der Vorteil dieser Kombination von Reaktionstechniken liegt in einer Minimierung von Apparatekosten.

### Beispiele

### Beispiele 1 bis 7

Aceton und Phenol wurden zu Bisphenol A umgesetzt in einem Gegenstromrohr von 30 cm Länge und 50 mm Durchmesser, das mit 160 g Katalysator befüllt war. Der Katalysator befand sich in zylindrischen Füllkörpern aus Maschendraht, welche ein freies Volumen von ca. 60% für den Gasstrom offen ließen und einen intensiven Stoffaustausch ermöglichten. Als Katalysator wurde ein sulfoniertes, mit 2 Gew.-% Divinylbenzol vernetztes Polystyrolharz (K1131, Bayer AG) eingesetzt, das mit 5 Mol-% 2,2-Dimethylthiazolidin modifiziert wurde.

### Beispiele 1 bis 3

Bei konstanter Eduktstromzusammensetzung wurde der Mengenstrom des Gaszulaufs variiert.

| Zusammensetzung | Aceton [Gew.-%] | Wasser [Gew.-%] | Phenol [Gew.-%] |
|---|---|---|---|
| Flüssigzulauf | 4,00 | 0,25 | 95,75 |
| Gaszulauf | 59,5 | 6,6 | 33,9 |

| **Beispiel** | **1** | **1a** | **2** | **2a** | **3** | **3a** |
|---|---|---|---|---|---|---|
| Kopftemperatur[°C] | 60,3 | 60,2 | 60,2 | 60,1 | 57,5 | 58,5 |
| Fußtemperatur[°C] | 63,1 | 62,9 | 63,1 | 63,0 | 63,3 | 63,1 |
| Druck [mbar] | 30 | 30 | 30 | 30 | 30 | 30 |
| Zulauf Kopf [g/h] | 600 | 600 | 600 | 600 | 600 | 600 |
| Zulauf Fuß [g/h] | 600 | 600 | 300 | 300 | 150 | 150 |
| Sumpf [g/h] | 690 | 688 | 640 | 650 | 651 | 649 |
| Destillat [g/h] | 485 | 495 | 250 | 240 | 102 | 100 |

Die Zusammensetzung des Rohprodukts unterhalb der Reaktionszone in diesem Gegenstromrohr ist in der folgenden Übersicht dargestellt. Hierbei ist zu beachten, daß in dieser einfachen Laborapparatur unterhalb der Reaktionszone keine destillative Abtrennung der Leichtsieder Aceton und Wasser erfolgt, so daß diese im Sumpfprodukt mit erscheinen. In den folgenden Analysen ergibt daher die Summe aller Sumpfprodukte (inclusive Aceton und Wasser) 100 %.

| **Analyse Sumpfprodukt** | **1** | **1a** | **2** | **2a** | **3** | **3a** |
|---|---|---|---|---|---|---|
| Phenol [Gew.-%] | 78,9 | 78,8 | 77,0 | 79,2 | 78,9 | 78,2 |
| Dimethylxanthen [Gew.-%] | 0,08 | 0,09 | 0,08 | 0,08 | 0,06 | 0,06 |
| o,o'-BPA [Gew.-%] | 0,013 | 0,014 | 0,015 | 0,014 | 0,013 | 0,013 |
| o,p'-BPA [Gew.-%] | 0,42 | 0,44 | 0,49 | 0,47 | 0,51 | 0,54 |
| p,p'-BPA [Gew.-%] | 13,3 | 13,5 | 15,5 | 14,9 | 15,1 | 15,6 |
| Chroman [Gew.-%] | 0,031 | 0,035 | 0,033 | 0,033 | 0,029 | 0,030 |
| BPA-Indan [Gew.-%] | 0,004 | 0,004 | 0,004 | 0,004 | 0,005 | 0,008 |
| Spiroindan [Gew.-%] | 0,021 | 0,023 | 0,022 | 0,021 | 0,020 | 0,020 |
| Trisphenol [Gew.-%] | 0,15 | 0,17 | 0,15 | 0,15 | 0,15 | 0,16 |
| Mol 402 [Gew.-%] | 0,001 | 0,001 | 0,007 | 0,006 | 0,001 | 0,001 |
| Σ unbek. Komp. [Gew.-%] | 0,016 | 0,019 | 0,080 | 0,040 | 0,040 | 0,050 |
| Aceton [Gew.-%] | 6,7 | 6,6 | 6,3 | 5,0 | 4,9 | 5,0 |
| Wasser [Gew.-%] | 0,36 | 0,31 | 0,29 | 0,08 | 0,27 | 0,33 |

| **Beispiel** | **1** | **1a** | **2** | **2a** | **3** | **3a** |
|---|---|---|---|---|---|---|
| Selektivität für p,p'-BPA [%] | 94,8 | 94,4 | 94,6 | 94,7 | 94,8 | 94,7 |
| Raum-Zeit-Ausbeute [g (BPA) /ml (Kat)·h] | 0,43 | 0,43 | 0,46 | 0,45 | 0,46 | 0,47 |
| Raum-Zeit-Ausbeute [g (BPA) /g (Kat)·h] | 0,57 | 0,57 | 0,61 | 0,60 | 0,61 | 0,63 |

### Beispiele 4 bis 7

Die Wasserkonzentration in der flüssigen Phase und die Reaktionstemperatur wurden variiert.

| **Beispiel** | **4** | | **5** | | **6** | | **7** | |
|---|---|---|---|---|---|---|---|---|
| | Flüssigzulauf | Gaszulauf | Flüssigzulauf | Gaszulauf | Flüssigzulauf | Gaszulauf | Flüssigzulauf | Gaszulauf |
| Aceton [Gew.-%] | 4,0 | 57,8 | 4,0 | 59,5 | 4,0 | 60,30 | 4,0 | 60,8 |
| Wasser [Gew.-%] | 0,50 | 12,5 | 0,25 | 6,6 | 0,10 | 2,7 | 0,00 | 0,00 |
| Phenol [Gew.-%] | 95,50 | 29,7 | 95,75 | 33,9 | 95,90 | 36,9 | 96,00 | 39,2 |

| **Beispiel** | **4** | **4a** | **5** | **5a** | **6** | **7** |
|---|---|---|---|---|---|---|
| Kopftemperatur[°C] | 56,2 | 56,2 | 60,2 | 60,1 | 63,5 | 65,7 |
| Fußtemperatur[°C] | 58,8 | 58,7 | 63,1 | 63,0 | 66,1 | 68,5 |
| Druck [mbar] | 30 | 30 | 30 | 30 | 30 | 30 |
| Zulauf Kopf [g/h] | 600 | 600 | 600 | 600 | 600 | 600 |
| Zulauf Fuß [g/h] | 300 | 300 | 300 | 300 | 300 | 300 |
| Sumpf [g/h] | 660 | 655 | 640 | 650 | 680 | 670 |
| Destillat [g/h] | 235 | 238 | 250 | 240 | 220 | 225 |

Die Zusammensetzung des Rohprodukts unterhalb der Reaktionszone in diesem Gegenstromrohr ist in der folgenden Übersicht dargestellt. Hierbei ist zu beachten. daß in dieser einfachen Laborapparatur unterhalb der Reaktionszone keine destillative Abtrennung der Leichtsieder Aceton und Wasser erfolgt, so daß diese im Sumpfprodukt mit erscheinen. In den folgenden Analysen ergibt daher die Summe der Sumpfprodukte (inclusive Aceton und Wasser) 100 %.

| **Analyse Sumpfprodukt** | **4** | **4a** | **5** | **5a** | **6** | **7** |
|---|---|---|---|---|---|---|
| Phenol [Gew.-%] | 81,6 | 82,0 | 77,0 | 79,2 | 75,5 | 74,4 |
| Dimethylxanthen [Gew.-%] | 0,05 | 0,05 | 0,08 | 0,08 | 0,09 | 0,09 |
| o,o'-BPA [Gew.-%] | 0,012 | 0,012 | 0,015 | 0,014 | 0,015 | 0,017 |
| o,p'-BPA [Gew.-%] | 0,32 | 0,33 | 0,49 | 0,47 | 0,61 | 0,72 |
| p,p'-BPA [Gew.-%] | 11,7 | 11,5 | 15,5 | 14,9 | 17,2 | 18,8 |
| Chroman [Gew.-%] | 0,018 | 0,018 | 0,033 | 0,033 | 0,046 | 0,068 |
| BPA-Indan [Gew.-%] | 0,003 | 0,000 | 0,004 | 0,004 | 0,011 | 0,024 |
| Spiroindan [Gew.-%] | 0,012 | 0,010 | 0,022 | 0,021 | 0,037 | 0,058 |
| Trisphenol [Gew.-%] | 0,10 | 0,09 | 0,15 | 0,15 | 0,22 | 0,29 |
| Mol 402 [Gew.-%] | 0,001 | 0,001 | 0,007 | 0,006 | 0,007 | 0,009 |
| Σunbek. Komp. [Gew.-%] | 0,02 | 0,02 | 0,08 | 0,04 | 0,04 | 0,05 |
| Aceton [Gew.-%] | 5,6 | 5,5 | 6,3 | 5,0 | 5,9 | 5,3 |
| Wasser [Gew.-%] | 0,50 | 0,50 | 0,29 | 0,08 | 0,25 | 0,16 |

| **Beispiel** | **4** | **4a** | **5** | **5a** | **6** | **7** |
|---|---|---|---|---|---|---|
| Selektivität für p,p'-BPA [%] | 95,6 | 95,6 | 94,6 | 94,7 | 94,2 | 93,4 |
| Raum-Zeit-Ausbeute [g (BPA) /ml (Kat)·h] | 0,36 | 0,35 | 0,46 | 0,45 | 0,55 | 0,59 |
| Raum-Zeit-Ausbeute [g (BPA) /g (Kat)·h] | 0,48 | 0,47 | 0,61 | 0,60 | 0,73 | 0,79 |

### Beispiele 8 bis 10

Die Versuchsapparatur und -durchführung entspricht derjenigen in den Versuchsbeispielen 1-7. In dieser Reihe wurden andere Katalysatoren eingesetzt. Alle Katalysatoren waren mit 5 Mol-% 2,2-Dimethylthiazolidin modifiziert.

Die Zusammensetzung der Eduktströme blieb in dieser Beispielserie konstant:

| Zusammensetzung | Aceton [Gew.-%] | Wasser [Gew.-%] | Phenol [Gew.-%] |
|---|---|---|---|
| Flüssigzulauf | 4,00 | 0,5 | 95,5 |
| Gaszulauf | 57,8 | 12,5 | 29,7 |

| **Beispiel** | **8** | **9** | **10** |
|---|---|---|---|
| Kopftemperatur[°C] | 56,2 | 56,2 | 56,2 |
| Fußtemperatur[°C] | 58,8 | 58,8 | 58,8 |
| Druck [mbar] | 30 | 30 | 30 |
| Zulauf Kopf [g/h] | 600 | 540 | 600 |
| Zulauf Fuß [g/h] | 300 | 270 | 300 |
| Sumpf [g/h] | 685 | 620 | 700 |
| Destillat [g/h] | 215 | 180 | 200 |
| Katalysator | Lewatit K 1221 Bayer | Amberlyst A 31 Röhm & Haas | Lewatit K 1431 Bayer |
| Einsatz Kat (g) | 200 | 180 | 200 |

Die Zusammensetzung des Rohprodukts unterhalb der Reaktionszone in diesem Gegenstromrohr ist in der folgenden Übersicht dargestellt. Hierbei ist zu beachten, daß in dieser einfachen Laborapparatur unterhalb der Reaktionszone keine destillative Abtrennung der Leichtsieder Aceton und Wasser erfolgt, so daß diese im Sumpfprodukt mit erscheinen. In den folgenden Analysen ergibt daher die Summe aller Sumpfprodukte (inclusive Aceton und Wasser) 100 %.

| **Analyse Sumpfprodukt** | **8** | **9** | **10** |
|---|---|---|---|
| Phenol [Gew.-%] | 80,0 | 85,0 | 87,1 |
| Dimethylxanthen [Gew.-%] | 0,00 | 0,05 | 0,00 |
| o,o'-BPA [Gew.-%] | 0,013 | 0,010 | 0,006 |
| o,p'-BPA [Gew.-%] | 0,53 | 0,37 | 0,21 |
| p,p'-BPA [Gew.-%] | 12,3 | 9,6 | 7,1 |
| Chroman [Gew.-%] | 0,044 | 0,027 | 0,020 |
| BPA-Indan [Gew.-%] | 0,006 | 0,004 | 0,008 |
| Spiroindan [Gew.-%] | 0,014 | 0,013 | 0,023 |
| Trisphenol [Gew.-%] | 0,051 | 0,063 | 0,045 |
| Mol 402 [Gew.-%] | 0,007 | 0,005 | 0,000 |
| Σ unbek. Komp. [Gew.-%] | 0,047 | 0,033 | 0,039 |
| Aceton [Gew.-%] | 6,3 | 4,3 | 4,8 |
| Wasser [Gew.-%] | 0,55 | 0,54 | 0,61 |

| **Beispiel** | **8** | **9** | **10** |
|---|---|---|---|
| Selektivität für p,p'-BPA [%] | 94,0 | 94,4 | 94,7 |
| Raum-Zeit-Ausbeute [g (BPA) /g (Kat)·h] | 0,42 | 0,33 | 0,25 |

## Patentansprüche

1. Verfahren zur Herstellung von Bis(4-hydroxyaryl)alkanen durch sauer katalysierte Umsetzung aromatischer Hydroxyverbindungen mit Ketonen, bei dem
eine Flüssigphase, die aromatische Hydroxyverbindung, Keton und gegebenenfalls Wasser enthält, und
eine Gasphase, die aromatische Hydroxyverbindung, Keton und gegebenenfalls Wasser in solchen Konzentrationen enthält, daß im Reaktor Wasser aus der Flüssigphase in die Gasphase und Keton aus der Gasphase in die Flüssigphase übergehen,
durch einen Reaktor geleitet werden.

2. Verfahren gemäß Anspruch 1, bei dem Gas- und Flüssigphase im Gegenstrom durch den Reaktor geleitet werden.

3. Verfahren gemäß Anspruch 1 oder 2, bei dem die Konzentrationen an aromatischer Hydroxyverbindung, Keton und gegebenenfalls Wasser in der dem Reaktor zugeführten Gasphase denen entsprechen, die in einer Gasphase vorliegen, die sich mit der dem Reaktor zugeführten Flüssigphase unter den im Reaktor herrschenden Temperatur- und Druckbedingungen im thermodynamischen Gleichgewicht befindet.

4. Verfahren gemäß Anspruch 1 oder 2, bei dem die Konzentrationen an aromatischer Hydroxyverbindung, Keton und gegebenenfalls Wasser in der dem Reaktor zugeführten Gasphase und der dem Reaktor zugeführten Flüssigphase so gewählt werden, daß die Temperatur und/oder die Konzentrationen an aromatischer Hydroxyverbindung, Keton und Wasser in der Flüssigphase nicht über die gesamte Länge des Reaktor konstant sind.

5. Verfahren gemäß Anspruch 1 oder 2, bei dem der Reaktor einen heterogenen sauren Katalysator enthält.

6. Verfahren gemäß Anspruch 5, bei dem als Katalysator ein saures Ionentauscherharz verwendet wird.

7. Verfahren gemäß Anspruch 1 oder 2, bei dem der Katalysator gemeinsam mit schwefelhaltigen Cokatalysatoren eingesetzt wird.

8. Verfahren gemäß Anspruch 1 oder 2, bei dem die Temperatur im Reaktor höher als die Kristallisationstemperatur des Reaktionsgemischs ist und maximal 130°C beträgt.

9. Verfahren gemäß Anspruch 1 oder 2, bei dem der Druck im Reaktor 5 mbar bis 1000 mbar beträgt.

10. Verfahren gemäß Anspruch 1 oder 2, bei dem die aromatische Hydroxyverbindung Phenol ist.

11. Verfahren gemäß Anspruch 1 oder 2, bei dem das Keton Aceton ist.

12. Verfahren gemäß Anspruch 1 oder 2, bei dem als Reaktor eine Reaktivrektifikationskolonne verwendet wird.

13. Verfahren gemäß Anspruch 1 oder 2, bei dem die dem Reaktor zugeführte Flüssigphase aromatische Hydroxyverbindung, Keton und daraus gebildete Reaktionsprodukte enthält

## Claims

1. A process for preparing bis(4-hydroxyaryl)alkanes by the acid catalysed reaction of aromatic hydroxy compounds with ketones, in which
a liquid phase, which contains the aromatic hydroxy compound, ketone and optionally water, and
a gas phase which contains the aromatic hydroxy compound, ketone and optionally water in concentrations such that, in the reactor, water from the liquid phase passes into the gas phase and ketone from the gas phase passes into the liquid phase,
are passed through a reactor.

2. A process according to Claim 1, in which the gas phase and the liquid phase are passed through the reactor in counterflow.

3. A process according to Claim 1 or 2, in which the concentrations of aromatic hydroxy compound, ketone and optionally water in the gas phase supplied to the reactor correspond to those which are present in a gas phase which is in thermodynamic equilibrium with the liquid phase supplied to the reactor under the temperature and pressure conditions prevailing in the reactor.

4. A process according to Claim 1 or 2, in which the concentrations of aromatic hydroxy compound, ketone and optionally water in the gas phase supplied to the reactor and in the liquid phase supplied to the reactor are chosen in such a way that the temperature and/or concentrations of aromatic hydroxy compound, ketone and water in the liquid phase are not constant over the entire length of the reactor.

5. A process according to Claim 1 or 2, in which the reactor contains a heterogeneous acid catalyst.

6. A process according to Claim 5, in which an acid ion exchange resin is used as the catalyst.

7. A process according to Claim 1 or 2, in which the catalyst is used together with sulfur-containing cocatalysts.

8. A process according to Claim 1 or 2, in which the temperature in the reactor is higher than the temperature of crystallisation of the reaction mixture and is at most 130°C.

9. A process according to Claim 1 or 2, in which the pressure in the reactor is 5 mbar to 1000 mbar.

10. A process according to Claim 1 or 2, in which the aromatic hydroxy compound is phenol.

11. A process according to Claim 1 or 2, in which the ketone is acetone.

12. A process according to Claim 1 or 2, in which a reactive rectification column is used as the reactor.

13. A process according to Claim 1 or 2, in which the liquid phase supplied to the reactor contains aromatic hydroxy compound, ketone and reaction products formed therefrom.

## Revendications

1. Procédé de préparation de bis(4-hydroxy-aryl)alcanes par réaction sous catalyse acide de composés hydroxylés aromatiques avec des cétones, dans lequel une phase liquide, qui contient le composé hydroxylé aromatique, la cétone et le cas échéant, l'eau, et une phase gazeuse, qui contient le composé hydroxylé aromatique, la cétone et le cas échéant, l'eau en des concentrations telles que dans le réacteur, l'eau passe de la phase liquide à la phase gazeuse et la cétone de la phase gazeuse à la phase liquide, sont conduites au travers d'un réacteur.

2. Procédé suivant la revendication 1, dans lequel les phases gazeuse et liquide sont conduites au travers du réacteur à contre-courant.

3. Procédé suivant la revendication 1 ou 2, dans lequel les concentrations en composé hydroxylé aromatique, en cétone et le cas échéant, en eau dans la phase gazeuse amenée dans le réacteur correspondent à celles qui se présentent dans une phase gazeuse qui se trouve en équilibre thermodynamique avec la phase liquide amenée dans le réacteur, dans les conditions de température et de pression régnant dans le réacteur.

4. Procédé suivant la revendication 1 ou 2, dans lequel les concentrations en composé hydroxylé aromatique, en cétone et le cas échéant, en eau dans la phase gazeuse amenée dans le réacteur et dans la phase liquide amenée dans le réacteur sont choisies de sorte que la température et/ou la concentration en composé hydroxylé aromatique, en cétone et en eau ne soient pas constantes dans la phase liquide sur toute la longueur du réacteur.

5. Procédé suivant la revendication 1 ou 2, dans lequel le réacteur contient un catalyseur acide hétérogène.

6. Procédé suivant la revendication 5, dans lequel on utilise comme catalyseur, une résine échangeuse d'ions.

7. Procédé suivant la revendication 1 ou 2, dans lequel le catalyseur est mis en oeuvre avec un cocatalyseur soufré.

8. Procédé suivant la revendication 1 ou 2, dans lequel la température dans le réacteur est supérieure à la température de cristallisation du mélange réactionnel et s'élève à 130°C au maximum.

9. Procédé suivant la revendication 1 ou 2, dans lequel la pression dans le réacteur se situe dans l'intervalle allant de 5 mbar à 1000 mbar.

10. Procédé suivant la revendication 1 ou 2, dans lequel le composé hydroxylé aromatique est le phénol.

11. Procédé suivant la revendication 1 ou 2, dans lequel la cétone est l'acétone.

12. Procédé suivant la revendication 1 1 ou 2, dans lequel on utilise comme réacteur, une colonne réactive de rectification.

13. Procédé suivant la revendication 1 ou 2, dans lequel la phase liquide amenée dans le réacteur contient le composé hydroxylé aromatique, la cétone et le produit de réaction formé à partir de ceux-ci.
